(19) **Europäisches Patentamt / European Patent Office / Office européen des brevets**

(11) **EP 1 469 317 B1**

(12) **EUROPEAN PATENT SPECIFICATION**

(45) Date of publication and mention of the grant of the patent:
**08.10.2008 Bulletin 2008/41**

(51) Int Cl.:
*G01N 33/86* (2006.01)        *G01N 33/92* (2006.01)
*G01N 33/564* (2006.01)

(21) Application number: **04007158.1**

(22) Date of filing: **25.03.2004**

(54) **Reagent for detecting anti-phospholipid antibody**

Reagenz zum Nachweis von anti-Phospholipid Antikörper

Réactif pour détecter anti-phospholipid anticorps

(84) Designated Contracting States:
**DE FR GB IT SE**

(30) Priority: **28.03.2003 JP 2003091987**

(43) Date of publication of application:
**20.10.2004 Bulletin 2004/43**

(73) Proprietor: **SYSMEX CORPORATION**
**Hyogo 651-0073 (JP)**

(72) Inventors:
- **Okuda, Masahiro,**
  **Sysmex Corporation**
  **Kobe-shi,**
  **Hyogo 651-0073 (JP)**
- **Muneo, Kenji,**
  **Sysmex Corporation**
  **Kobe-shi,**
  **Hyogo 651-0073 (JP)**

(74) Representative: **HOFFMANN EITLE**
**Patent- und Rechtsanwälte**
**Arabellastrasse 4**
**81925 München (DE)**

(56) References cited:
WO-A-01/21183        US-B1- 6 261 792
US-B1- 6 417 004     US-B1- 6 451 610

- **JACOBSEN E M ET AL: "The evaluation of clotting times in the laboratory detection of lupus anticoagulants" THROMBOSIS RESEARCH, TARRYTOWN, NY, US, vol. 104, no. 4, 15 November 2001 (2001-11-15), pages 275-282, XP002261843 ISSN: 0049-3848**
- **SAKAKURA M ET AL: "Coagulation tests and anti-phospholipid antibodies in patients positive for lupus anticoagulant." CLINICAL AND APPLIED THROMBOSIS/HEMOSTASIS : OFFICIAL JOURNAL OF THE INTERNATIONAL ACADEMY OF CLINICAL AND APPLIED THROMBOSIS/HEMOSTASIS. JUL 2000, vol. 6, no. 3, July 2000 (2000-07), pages 144-150, XP008033930 ISSN: 1076-0296**
- **DATABASE WPI Section Ch, Week 199334 Derwent Publications Ltd., London, GB; Class B04, AN 1993-267048 XP002292715 & JP 05 180835 A (SRL KK) 23 July 1993 (1993-07-23)**
- **NORBIS FRANCESCA ET AL: "Diluted Russell's viper venom time and colloidal silica clotting time for the identification of the phospholipid-dependent inhibitors of coagulation" THROMBOSIS RESEARCH, vol. 85, no. 5, 1997, pages 427-431, XP002292713 ISSN: 0049-3848**
- **SIBILIA JEAN: "Antiphospholipid syndrome: why and how should we make the diagnosis?" JOINT, BONE, SPINE : REVUE DU RHUMATISME. MAR 2003, vol. 70, no. 2, March 2003 (2003-03), pages 97-102, XP002292714 ISSN: 1297-319X**
- **"Webster's New Dictionary of the English Language" POPULAR PUBLISHING , NEW YORK * page 114 ***

**Description**

**BACKGROUND OF THE INVENTION**

Field of the Invention

The present invention relates to a coagulation time reagent used in diagnosing anti-phospholipid syndrome (APS) in the fields such as clinical chemistry and pharmaceutical research, and a reagent kit using the reagent, and more particularly to a reagent capable of precisely determining patients positive in lupus anticoagulant by a blood test in vitro and quantitatively detecting lupus anticoagulant, a reagent kit using the reagent, and a method for detecting an anti-phospholipid antibody.

Discussion of the Related Art

Anti-phospholipid antibody is a generic term for autoantibodies against phospholipids having negative charges, such as cardiolipin, phosphatidylserine, and phosphatidic acid. Lupus anticoagulant is one of the examples of the anti-phospholipid antibodies. Anti-phospholipid syndrome (APS) is a generic term for diseases in which one of these autoantibodies is positive, and which show specific clinical symptoms such as thrombosis, miscarriage, premature.labor, and thrombocytopenia.

A recent study revealed that LA, which is one of causative substances of APS, is a heterogeneous antibody and that its antigen is an antibody against a complex of a negatively-charged phospholipid (phosphatidylserine) and β 2-glycoprotein I (β2-GPI) as a cofactor, or a complex of a negatively-charged phospholipid and prothrombin. It is conceived that LA shows anticoagulation because binding of LA to the complex inhibits prothrombin from converting into thrombin. Further, there is known that LA is immunoglobulin that inhibits phospholipid-dependent coagulation without suppressing individual activities of coagulating factors (Guidebook on Laboratory Tests, Kanehara & Co., Ltd., 31st Edition, p442).

There have been known reagents containing a certain amount of phospholipid for detecting LA having the aforementioned properties such as cephalin derived from rabbit brain, cephalin derived from bovine brain, soy bean lecithin, and viper venom. If LA exists in a blood sample, the LA neutralizes the phospholipid in the reagent, which gives rise to shortage of the phospholipid necessary for initiating cascade reaction of coagulating the blood. As a result of such shortage, the coagulation time of each of the blood samples is prolonged in proportion to the amount of phospholipid in the reagent which has been neutralized by LA. Thus, this approach makes it possible to determine whether each blood sample is LA positive or not.

In addition to the above approach, another approach has been conducted, in which two kinds of reagents having different concentrations of phospholipid from each other are combined and in which the diluted reagent is employed. Dilution of the reagent may secure detection accuracy in light of the fact that the less the phospholipid in the reagent, the higher the detection sensitivity of LA is.

As an example of the latter approach, there is known a technique of judging whether a blood sample is LA-positive or not by using an activated partial thromboplastin time reagent (hereinafter, simply called as "APTT reagent"), which is a combination of two kinds of reagents having different concentrations of phospholipid from each other, and by calculating Rosner Index or Lupus Ratio (LR) based on the coagulation times respectively obtained with use of the two kinds of reagents.

Examples of commercially available coagulation time reagents are a reagent based on diluted Russel's viper venom, called Gradipore LA (Gradipore Ltd., Australia), and a reagent based on Staclot LA using hexagonal phosphatidyl ethanolamine (Roche Diagnostics K.K.).

According to a known scientific report (V.Chantarangkul, et al., Thromb. Res. 1992, 67:355-365; E.Rosner, et al., Thromb. Haemost. 1987, 57:144-149), there has been practiced a method of detecting LA based on a difference in concentration of phospholipids derived from rabbit brain, for example.

The above methods are either a screening method or a qualitative method. Further, the reagents used in the above methods are not only specifically reacted to anti-phospholipid antibodies or lupus anticoagulant but also reacted to other component or components. Accordingly, there is likelihood that a sample may be misjudged as LA positive contrary to the fact that the sample is not LA positive, or conversely, the sample may be misjudged as LA negative contrary to the fact that the sample is LA positive if the sample is derived from a patient having low responsiveness to the reagent.

In view of the above, there are demanded a reagent and a detecting method which are capable of quantitatively or specifically detecting an anti-phospholipid antibody or immunoglobulin derived from lupus anticoagulant to raise precision in diagnosing.

As a quantitative method of detecting anti-phospholipid antibody, an immunological method in which an amount of modified cardiolipin is measured in the presence of β2-GPI has been developed. This method is not a direct one of measuring an amount of anti-cardiolipin antibody.

In the above circumstances, Brandt et al. in the International Society on Thrombosis and Hemostasis (ISTH) prepared a guideline (Thromb Haemost 1995; 74(4): 1185-90) regarding lupus anticoagulant, and established the Sapporo Criteria (Arthritis Rheum 1999; 42(7):1309-11) for diagnosing APS. The guideline and the criteria recommend that a test based on combination of two or three different kinds of determining principles should be conducted for diagnosing APS or LA, in place of conducting a test based on a single determining principle.

There has been reported a method for precisely detecting phosphatidylserine-dependent anti-prothrombin antibody by utilizing specific properties of the antibody. Since this method is an immunological detecting method (Arthritis Rheum 2000;43(9):1982-93), the detection is not easy, as compared to a coagulation test.

## BRIEF DESCRIPTION OF THE DRAWINGS

FIG. 1 is a graph showing examination results in Example 2, namely, a relation between concentration of HBR and a coagulation time with respect to each sample; and

FIG. 2 is a graph showing examination results in Example 3, namely an affecting ratio to a coagulation time in each serum from different kinds of animals.

## SUMMARY OF THE INVENTION

An object of the present invention is to provide a reagent capable of precisely detecting an anti-phospholipid antibody, which is an agent for capturing APS, especially LA, by a relatively easy coagulation test judging based on a coagulation time, and a reagent kit using the same.

Another object of the present invention is to provide a method for precisely and easily detecting the anti-phospholipid antibody with use of the reagent kit.

Yet another object of the present invention is to provide a method for specifically and quantitatively determining the anti-phospholipid antibody in a sample.

The inventors found, as a result of scientific research and development from various aspects, that anti-phospholipid antibodies which is cause of APS, particularly LA, specifically binds antibodies or the like derived from vertebrate animals other than humans, such as mouse IgG, horse IgG, heterophilic blocking reagent (HBR), NMS, MAK33, mouse serum, and horse serum, and that the binding of LA to the antibody or the like hinders the binding of LA to phospholipids in a coagulation time reagent, and thus, accomplished the present invention.

Specifically, according to one aspect, the present invention comprises a coagulation time reagent according to claims 1-4.

According to another aspect of the present invention, a reagent kit detecting anti-phospholipid antibody comprises the inventive coagulation time reagent (first coagulation time reagent), and a second coagulation time reagent which contains the composition for coagulation in the absence of the antibodies, the plasma, the serum, and the immunoglobulin derived from the vertebrate animals other than the humans.

According to yet another aspect of the present invention, a method for detecting the anti-phospholipid antibody comprises: a step of contacting a sample with the first coagulation time reagent and the second coagulation time reagent respectively; and a step of comparing a first coagulation time of the sample exposed to the first coagulation time reagent, with a second coagulation time of the sample exposed to the second coagulation time reagent.

Accordingly, also described herein is a method for measuring the anti-phospholipid antibody comprises: a step of contacting a sample with one labeled component selected from the group consisting of antibodies, plasma, serum, and immunoglobulin derived from vertebrate animals other than humans; and a step of measuring the quantity of the labeled non-human-derived antibody or the like bound to the sample.

## DETAILED DESCRIPTION OF THE INVENTION

[Coagulation Time Reagent]

The coagulation time reagent of the invention contains a composition for coagulation; and at least one component selected from the group consisting of antibodies, plasma, serum, and immunoglobulin derived from vertebrate animals other than humans.

The composition for coagulation throughout the specification and claims is a composition necessary for causing coagulation in vitro, and includes: calcium ions; phospholipids; and at least one component selected from the group consisting of an activator, viper venom, and a tissue factor.

Examples of the phospholipids are phosphatidylserine, phosphatidylethanolamine, and phosphatidylcholine, including combination of two or more kinds thereof.

Preferable example of the activator includes kaolin, celite, silica, and ellagic acid. One or a combination of these activators may be used. The viper venom is preferably at least one selected from the group consisting of Russel's viper venom, textarin venom, and ecarin venom. The tissue factor may be one derived from rabbit brain, derived from human placenta, or a recombinant.

The activator, the viper venom, or the tissue factor may be optionally selected depending on the kind of the coagulation time reagent. For instance, in case that the reagent contains calcium ions, phospholipids, and an activator, the reagent functions as an activated partial thromboplastin time (APTT) reagent. In case that the reagent contains calcium ions, phospholipids, and viper venom, the reagent functions as a Russel's viper venom time (RVVT) reagent. In case that the reagent contains calcium ions, phospholipids, and a tissue factor, the reagent functions as a prothrombin time (PT) reagent.

The content of the composition for coagulation in the inventive reagent can trigger coagulation in blood or plasma. The content of the composition for coagulation may be optionally arranged considering a mixing ratio of the reagent to the sample to be measured.

The at least one component selected from the group consisting of the antibodies, the plasmas, the serum, and the immunoglobulin derived from the vertebrate animals other than the humans (hereinafter, called as "non-human-derived antibody or the like", unless otherwise specifically mentioned) is contained in the inventive reagent in order to capture the anti-phospholipid antibody in the sample to be measured. Therefore, in case that blood coagulation is triggered with use of the coagulation time reagent of the present invention, the anti-phospholipid antibody to be detected, namely, lupus anticoagulant derived from humans (human-derived LA) is captured by the non-human-derived antibody or the like and prevented from binding phospholipids in the reagent. In other words, in case of the coagulation time measured by use of the inventive coagulation time reagent, existence of anti-phospholipid antibody can be ignored because extension in coagulation time resulting from the existence of the anti-phospholipid antibody can be prevented.

Preferred examples of the non-human-derived antibody or the like to be contained in the inventive reagent are antibodies, plasmas, serum or immunoglobulin derived from mammals other than humans and swine. Specifically, mouse IgG, horse IgG, bovine IgG, mouse serum, horse serum, bovine serum, and α-globulin derived from mouse, horse, or bovine may be used as the non-human-derived antibody or the like. Heterophilic blocking reagent (HBR) by Scantibodies Laboratory, Inc., MAK33 by Roches Diagnostics K.K., and NM8 are examples of the commercially available products. HBR has been known as an agent to be added to a sample for the purpose of blocking interference by a heterophilic antibody in the typical immunological method. Further, the inventors found that HBR specifically binds to anti-phospholipid antibodies, particularly LA, and yet does not bind to other coagulation-time related component except the anti-phospholipid antibodies in a blood sample. Thus, HBR may be used as an agent for capturing the anti-phospholipid antibodies in the inventive reagent.

The non-human-derived antibody or the like may be an antibody derived from a single kind of animal, or may be an antibody derived from two or more kinds of animals, e.g., a mixture of mouse IgG and horse IgG.

The content of the non-human-derived antibody or the like in the inventive coagulation time reagent may preferably be in the range from 0.1 to 50 mg per ml of a sample to be measured, more preferably, in the range from 0.2 to 10 mg, further preferably, in the range from 0.5 to 6 mg. If the content of the non-human-derived antibody or the like is not greater than 0.1 mg per 1ml of a sample derived from an APS patient, binding of all the anti-phospholipid antibodies to the non-human-derived antibody or the like is not secured, and therefore, extension in coagulation time may be observed due to the existence of the non-bound anti-phospholipid antibodies.

If necessary, the inventive coagulation time reagent may contain a buffer such as HEPES and Tris buffer, in addition to the above components (i.e. a composition for coagulation and a non-human-derived antibody or the like). The concentration of the buffer may be a concentration generally used in the field of clinical chemistry, and is determined empirically based on simplified and repeated experiments. The inventive reagent may contain a protein such as α-globulin.

The inventive reagent may be accommodated in a single container as a mixture, or may be accommodated in separate containers, as a combination of a first preparatory reagent and a second preparatory reagent. In the latter case, the first preparatory reagent contains at least one component selected from the group consisting of antibodies, plasmas, serum, and immunoglobulin derived from vertebrate animals other than humans; phospholipids; and at least one component selected from the group consisting of an activator, viper venom, and a tissue factor, and the second preparatory reagent contains calcium ions.

[Reagent kit]

The reagent kit of the invention is a reagent kit for precisely detecting anti-phospholipid antibodies in blood or blood plasma derived from subjects, particularly lupus anticoagulant derived from humans (human LA). The human LA may be of any class including IgG, IgM, and IgA.

The inventive reagent kit comprises the inventive coagulation time reagent (also called as "first coagulation time reagent"), and a second coagulation time reagent which contains the composition for coagulation in the absence of non-human-derived antibody or the like. In other words, the inventive reagent kit is a combination of two different kinds of coagulation time reagents which differ from each other in the point as to whether the coagulation time reagent contains the non-human-derived antibody or the like, as the agent for capturing the anti-phospholipid antibodies.

The composition for coagulation in the first coagulation time reagent, and the composition for coagulation in the second coagulation time reagent should be of a kind or identical to each other. For instance, in the case where the composition for coagulation in the first coagulation time reagent is APTT reagent, the composition for coagulation in the second coagulation time reagent is also APTT reagent. Furthermore, it is preferable that the constitutions of the first and second coagulation time reagents are identical to each other.

In the case where the first coagulation time reagent is a combination of the first preparatory reagent and the second preparatory reagent, the second coagulation time reagent may preferably be a combination of two different kinds of preparatory reagents, namely, a third preparatory reagent and a fourth preparatory reagent. In this case, the third preparatory reagent corresponding to the first preparatory reagent contains phospholipids; and at least one component selected from the group consisting of an activator, viper venom, and a tissue factor, and the fourth preparatory reagent corresponding to the second preparatory reagent contains calcium ions.

Since the second coagulation time reagent does not contain the non-human-derived antibodies or the like serving as the agent for capturing anti-phospholipid-antibody, if a sample derived from an APS patient is exposed to the second coagulation time reagent, the sample shows extension in coagulation time due to the existence of the anti-phospholipid antibody (e.g., LA). On the other hand, in the case where the sample is exposed to the first coagulation time reagent, extension in coagulation time due to the existence of the anti-phospholipid antibody (e.g. LA) is suppressed, even if the sample is derived from the APS patient, because the anti-phospholipid antibody is captured by the non-human-derived antibody or the like in the first coagulation time reagent. In this way, if a first coagulation time of a sample exposed to the first coagulation time reagent is significantly different from a second coagulation time of the sample exposed to the second coagulation time reagent, the sample is determined positive to the anti-phospholipid antibody, whereas if there is not a significant difference in coagulation time, the sample is determined negative to the anti-phospholipid antibody.

[Detecting method]

According to the detecting method of the present invention, the reagent kit described above is employed. Specifically, the inventive detecting method comprises: a step of contacting a sample with the first coagulation time reagent and the second coagulation time reagent respectively; and a step of comparing a first coagulation time of the sample exposed to the first coagulation time reagent, with a second coagulation time of the sample exposed to the second coagulation time reagent. If the first coagulation time is significantly different from the second coagulation time, it is judged that the sample contains an anti-phospholipid antibody, particularly LA.

It may be possible to calculate an affecting ratio to the coagulation time according to the following equation which uses the first coagulation time (t1) and the second coagulation time (t2) as parameters, so as to judge whether the sample contains an anti-phospholipid antibody.

$$\text{affecting ratio} = 1 - ((t1 - t2) / t1)$$

The affecting ratio obtained by the above calculation is substantially 1 in not only the normal plasma but also the sample containing anticoagulant such as heparin, although the coagulation time of the sample containing anticoagulant is extended. On the contrary, samples containing anti-phospholipid antibodies generally show an affecting ratio of not smaller than 1.2 depending on the content of the anti-phospholipid antibodies.

The sample to be measured may be blood or blood plasma collected from patients, or may be a mixture of the blood or the blood plasma with blood plasma (normal plasma) from a normal healthy individual. A blood-derived substance prepared for a general blood coagulation test may also be used as the sample. In view of this, plasma or serum obtained by isolation of a blood sample from a subject according to a conventional process such as centrifugal separation may be used. The blood sample of a subject may be isolated without pretreatment or with addition of an anticoagulant such as heparin. Even if the sample contains an anticoagulant, it is conceived that the anticoagulant substantially does not affect the first coagulation time, since the non-human-derived antibody or the like does not show affinity to the anticoagulant. In order to attain further precise detection while suppressing non-specific binding in the detecting method, a specific fraction obtained by further isolation of the plasma or the serum by ultra centrifugal separation may be used. It is preferable that a mixed solution of the sample and the first (second) coagulation time reagent has the pH from 6 to 8 and the concentration of the salt from 1 to 500mM.

It is preferable to contact the first coagulation time reagent with the sample for a time sufficient to securely bind of the non-human-derived antibody or the like contained in the first coagulation time reagent to the anti-phospholipid antibody. Specifically, the contacting time generally ranges from 1 to 30 minutes, preferably from 2 to 10 minutes, and more preferably about 5 minutes. It is preferable to carry out the contacting step at a temperature from 30 to 40°C.

[Application of immunological method]

It is possible to measure the anti-phospholipid antibody in a sample by using a non-human-derived antibody or the like capable of binding to the anti-phospholipid antibody.

Specifically, the quantity of the anti-phospholipid antibody (LA) is determined by contacting a sample with a solid phase in which a non-human-derived antibody or the like is fixed and then by measuring the quantity of the labeled non-human-derived antibody or the like which binds the anti-phospholipid antibody.

The method for measuring the anti-phospholipid antibody may be a single-step method or a two-step method. Either one of the methods may be applicable in the present invention.

According to the single-step method, prepared is a solid phase to which a non-human-derived antibody or the like (primary antibody) is fixed, and a sample and a labeled non-human-derived antibody or the like (labeled secondary antibody) are added. The mixture is incubated, e.g., at a temperature from 4 to 40°C for a period from 1 minute to 1 day to produce a complex of the primary antibody, sample (anti-phospholipid antibody), and labeled secondary antibody. The complex will be hereinafter referred to as "the primary antibody/sample (anti-phospholipid antibody)/labeled secondary antibody". After the labeled non-human-derived antibody or the like that did not constitute the complex is removed by rinsing, the quantity of the anti-phospholipid antibody is determined by measuring the quantity of the labeled non-human-derived antibody or the like in the complex.

According to the two-step method, prepared is a solid phase to which a non-human-derived antibody or the like (primary antibody) is fixed, followed by addition of a sample for incubation, e.g., at a temperature from 4 to 40°C for a period from 1 minute to 2 hours, and rinsing. Next, a labeled non-human-derived antibody or the like (labeled secondary antibody) is added for reaction with the sample which has been bound to the primary antibody. The reaction is conducted, for example, at a temperature from 4 to 40°C for a period from 1 minute to 2 hours. As a result of the reaction, a complex of the primary antibody/sample (anti-phospholipid antibody)/labeled secondary antibody is produced. The labeled secondary antibody that has not bound to the sample is removed by rinsing, and then the quantity of the anti-phospholipid antibody is determined by measuring the quantity of the labeled non-human-derived antibody or the like in the complex.

A micro titer plate or polystyrene beads may be used as the solid phase. For example, the non-human-derived antibody is fixed to the solid phase by dissolving the non-human-derived antibody or the like in a buffer, putting the solid phase in the solution to contact the non-human-derived antibody with the solid phase, and letting the solution stand at 4°C overnight or longer. Since it is not expected that the entirety of the non-human-derived antibody or the like is securely and uniformly coated over the solid phase, it is preferable to dissolve bovine serum albumin in a Tris buffer and to contact the solution with the solid phase for coating the albumin over the solid phase.

It may be possible to use a non-labeled secondary antibody, in place of using the labeled secondary antibody, to form a complex of a primary antibody/sample (anti-phospholipid antibody)/non-labeled secondary antibody, and then to label the non-labeled secondary antibody in the complex with a label. For example, labeling is accomplished through a reaction between avidin and biotin.

Examples of the labeling component are enzymes such as alkaliphosphotase and peroxidase; fluorescent materials; chemiluminescent materials; and radioisotopes.

A known method for measuring the labeling component may be optionally selected depending on the kind of the labeling component. In case of labeling with an enzyme, the labeled antibody is quantitatively determined by measuring the enzyme activity. In case of labeling with a fluorescent material, the labeled antibody is quantitatively determined with use of a fluorometer. In case of labeling with a chemiluminescent material, the labeled antibody is quantitatively determined by a chemilumiescence technique using an enzyme. In case of labeling with a radioisotope, the labeled antibody is quantitatively determined with use of a scintillation counter. In case of utilizing a coloring substrate upon which an enzyme is acted, for instance, if the antibody is labeled with alkaliphosphotase, p-nitrophenylphosphate is used as the substrate, and if the antibody is labeled with peroxidase, 2'-adino-bi-(3'-ethylbenzylthiazoline sulphonate) is used as the substrate. In both of the cases, the labeled antibody is quantitatively determined based on an absorbance of an enzyme reaction product with use of a spectrophotometer.

**EXAMPLES**

The present invention will be further illustrated below by means of a number of concrete practical examples, which however do not in any way restrict the scope of the invention.

Example 1

(Process for preparing reagent)

50mM HEPES buffer, and 25mM TRIS buffer (pH7.35) were mixed with 0.1mM of ellagic acid solution with addition of 5 μg/ml of phosphatidylserine PS), 20 μg/ml of phosphatidylethanolamine (PE), and 70 μg/ml of phosphatidylcholine (PC) as phospholipids to prepare a third preparatory reagent of a second coagulation time reagent

A first preparatory reagent of the first coagulation time reagent was prepared by adding HBR to the third preparatory reagent, so that the concentration of the HBR was 400 μg/ml. HBR is a commercially available heterophilic blocking reagent manufactured by Scantibodies Laboratory, Inc., and the primary ingredient of the HBR was IgG derived from a mouse.

(Measuring method)

Normal plasma (COAGTROL N ® by Sysmex Corporation), plasma derived from LA-positive patients, plasma from patients administered with warfarin, plasma from patients administered with heparin, and plasma from patients having the factor VIII deficiency were used as samples to be tested. Commercially available products from NIBSC, George King Bio-medical, Inc., Gradipore Ltd., ADI, and SUNFCO Ltd. were used as the samples from the patients.

These samples were divided into two groups. 50 μl each of the samples in the one group was mixed with 50 μl of the first preparatory reagent of the first coagulation time reagent. 50 μl each of the samples in the other group was mixed with 50 μl of the third preparatory reagent of the second coagulation time reagent. Each mixture was heated to 37°C for 5 minutes. Thereafter, 50 μl of 25 mmol/l calcium chloride solution was added to each sample in the one group and the other group respectively. The calcium chloride solution corresponds to a second preparatory reagent of the first coagulation time reagent in the one group, and to a fourth preparatory reagent of the second coagulation reagent in the other group. Thus, coagulation was initiated in each sample in the two groups. The coagulation time was measured by an automatic blood coagulation analyzer "Coagrex-800" (available from Shimadzu Corp.). The measurement was conducted with respect to each sample twice. The measurement results are shown in Table 1. The affecting ratio is calculated to the following equation.

$$\text{affecting ratio} = 1 - ((t1 - t2) / t1)$$

wherein t1 is the first coagulation time when exposed to the first coagulation time reagent, and t2 is the second coagulation time when exposed to the second coagulation time reagent.

Table 1

| Test Sample | Coagulation time (sec) | | Affecting ratio |
|---|---|---|---|
| | Second coagulation time reagent (t2) | First coagulation time reagent (t1) | |
| Normal plasma 1 | 27.1 | 26.7 | 1.01 |
| Normal plasma 2 | 29.1 | 28.8 | 1.01 |
| Normal plasma 3 | 28.4 | 28.1 | 1.01 |
| LA-positive plasma 1 | 47.6 | 38.1 | 1.25 |
| LA-positive plasma 2 | 60.7 | 34.1 | 1.78 |
| LA-positive plasma 3 | 83.5 | 37.5 | 2.23 |
| LA-positive plasma 4 | 53.6 | 33.7 | 1.59 |
| LA-positive plasma 5 | 66.2 | 36.7 | 1.80 |
| Heparinized plasma 1 | 45.6 | 44.6 | 1.02 |
| Heparinized plasma 2 | 70.2 | 71.2 | 0.99 |
| Warfarinized plasma 1 | 65.2 | 66.6 | 0.98 |

(continued)

| Test Sample | Coagulation time (sec) | | Affecting ratio |
|---|---|---|---|
| | Second coagulation time reagent (t2) | First coagulation time reagent (t1) | |
| Warfarinized plasma 2 | 43.7 | 44.1 | 0.99 |
| Factor VIII deficiency | 80.3 | 81.2 | 0.99 |
| FactorIX -deficiency | 91.2 | 92.5 | 0.99 |

As is obvious from Table 1, the LA-containing plasma sample exposed to the first coagulation time reagent containing HBR showed a significantly shorter coagulation time (first coagulation time), compared with a coagulation time (second coagulation time) of the sample exposed to the second coagulation time reagent which does not contain HBR. The result reveals that selective binding of HBR to human LA suppresses extension in coagulation time.

On the other hand, the normal plasma, the heparinized plasma, the warfarinized plasma, the plasmas from the patients having the factor VIII deficiency and the factorIX deficiency did not show a significant difference between the first coagulation time and the second coagulation time. The results reveal that HBR does not affect the coagulation of normal plasma. Further, even if a coagulation time is extended in the samples added with the anticoagulant such as heparin and warfarin, and in the samples from the patients having a coagulant deficiency such as the factor VIII deficiency and the factor IX deficiency, a significant difference between the first coagulation time and the second coagulation time was not observed with respect to these samples, because the extension was deemed not to have been caused by the anti-phospholipid-antibodies.

Example 2

Prepared was a first coagulation time reagent having final HBR concentration as shown in Table 2 by adding HBR to Thrombocheck APTT-SLA (Sysmex Corporation) as a composition for coagulation. The Thrombocheck APTT-SLA is a commercially available APTT reagent having high sensitivity to LA. It should be noted that 0 $\mu$g regarding the HBR concentration in Table 2 means that the reagent corresponds to a second coagulation time reagent which does not contain HBR.

50 $\mu$l each of a mixture of plasma from a patient and normal plasma at a mixing ratio of 1:1 was used as a test sample. The coagulation time reagents each having a different HBR concentration were added to each of the samples, and coagulation times of the respective samples exposed to each of the coagulation time reagents where measured.

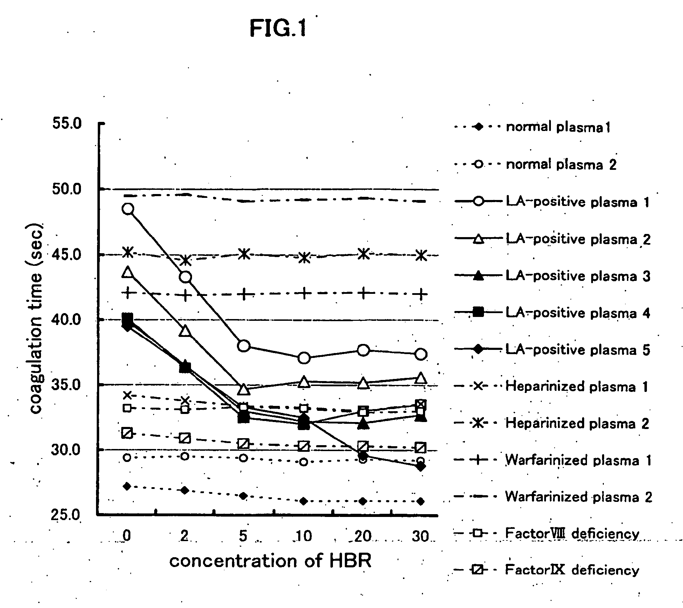

The measurement was conducted twice with respect to each sample by the automatic blood coagulation analyzer "Coagrex-800" (available from Shimadzu Corp.). The measurement results are shown in Table 2 and FIG. 1. An affecting ratio of the respective coagulation times (corresponding to the first coagulation times) of the samples each treated with the reagents showing the different HBR concentrations, relative to the respective coagulation times (corresponding to the second coagulation times) of the samples each treated with the reagent containing 0 $\mu$g in HBR concentration (i.e. the second coagulation time reagent), was calculated. The results of calculation are shown in Table 3.

Table 2

| Coagulation time (sec.) | | | | | | |
|---|---|---|---|---|---|---|
| Sample | concentration of HBR ($\mu$g/sample) | | | | | |
| | 0 | 2 | 5 | 10 | 20 | 30 |
| normal plasma 1 | 27.2 | 26.9 | 26.5 | 26.1 | 26.1 | 26.1 |
| normal plasma 2 | 29.4 | 29.5 | 29.4 | 29.1 | 29.3 | 29.2 |
| LA-positive plasma 1 | 48.5 | 43.3 | 38.0 | 37.1 | 337.7 | 37.4 |
| LA-positive plasma 2 | 43.7 | 39.2 | 34.7 | 35.3 | 35.2 | 35.6 |
| LA-positive plasma 3 | 39.9 | 36.5 | 33.0 | 32.2 | 32.1 | 32.7 |
| LA-positive plasma 4 | 40.1 | 36.3 | 32.5 | 32.0 | 33.0 | 33.5 |
| LA-positive plasma 5 | 39.5 | 36.4 | 33.3 | 32.5 | 29.6 | 28.8 |
| Heparinized plasma 1 | 34.2 | 33.8 | 33.4 | 33.3 | 33.0 | 33.6 |

(continued)

| Coagulation time (sec.) | | | | | | |
|---|---|---|---|---|---|---|
| Sample | concentration of HBR (μg/sample) | | | | | |
| | 0 | 2 | 5 | 10 | 20 | 30 |
| Heparinized plasma 2 | 45.2 | 44.6 | 45.1 | 44.8 | 45.1 | 45.0 |
| Warfarinized plasma 1 | 42.1 | 41.9 | 42.0 | 42.1 | 42.1 | 42.0 |
| Warfarinized plasma 2 | 49.5 | 49.6 | 49.1 | 49.2 | 49.3 | 49.1 |
| Factor VIII deficiency | 33.2 | 33.1 | 33.3 | 33.2 | 32.9 | 33.0 |
| FactorIX deficiency | 31.3 | 30.9 | 30.5 | 30.3 | 30.3 | 30.2 |

Table 3

| Affecting ratio | | | | | | |
|---|---|---|---|---|---|---|
| Sample | concentration of HBR (μ g/sample) | | | | | |
| | 0 | 2 | 5 | 10 | 20 | 30 |
| normal plasma 1 | 1.00 | 1.01 | 1.03 | 1.04 | 1.04 | 1.04 |
| normal plasma 2 | 1.00 | 1.00 | 1.00 | 1.01 | 1.00 | 1.01 |
| LA-positive plasma 1 | 1.00 | 1.12 | 1.28 | 1.31 | 1.29 | 1.30 |
| LA-positive plasma 2 | 1.00 | 1.11 | 1.26 | 1.24 | 1.24 | 1.23 |
| LA-positive plasma 3 | 1.00 | 1.09 | 1.21 | 1.24 | 1.24 | 1.22 |
| LA-positive plasma 4 | 1.00 | 1.10 | 1.23 | 1.25 | 1.22 | 1.20 |
| LA-positive plasma 5 | 1.00 | 1.09 | 1.19 | 1.22 | 1.33 | 1.37 |
| Heparinized plasma 1 | 1.00 | 1.01 | 1.02 | 1.03 | 1.04 | 1.02 |
| Heparinized plasma 2 | 1.00 | 1.01 | 1.00 | 1.01 | 1.00 | 1.00 |
| Warfarinized plasma 1 | 1.00 | 1.00 | 1.00 | 1.00 | 1.00 | 1.00 |
| Warfarinized plasma 2 | 1.00 | 1.00 | 1.01 | 1.01 | 1.00 | 1.01 |
| Factor VIII deficiency | 1.00 | 1.00 | 1.00 | 1.00 | 1.01 | 1.01 |
| FactorIX deficiency | 1.00 | 1.01 | 1.03 | 1.03 | 1.03 | 1.04 |

Table 2 and FIG. 1 reveal that the coagulation time is shortened depending on the HBR concentration with respect to the plasma containing human LA, and that the coagulation times of plasmas containing LA depend on the concentration of the non-human-derived antibody or HBR. On the other hand, there was not observed a significant difference in coagulation time due to the difference in concentration of the added HBR with respect to the normal plasma, the heparinized plasma, the warfarinized plasma, the plasma from the patients having the factor VIII deficiency and the factorIX deficiency. Further, FIG. 1 and Table 3 show that the affecting ratio is not smaller than 1.2 when not less than 5μg of HBR (corresponding to 0.1mg/ml in HBR concentration to the sample) was added to 50 μl of the sample from the LA-positive patient.

Example 3

Next, a relation between the kind of the vertebrate animal used in the experiments and its affinity to human LA was examined.

In this Example, prepared was a first coagulation time reagent containing 2mg/ml of serum each derived from the vertebrate animals as shown in Table 4, in place of HBR. The first coagulation time reagent and 5-times diluted plasma, as a test sample, were mixed at a mixing ratio of 9:1. Plasmas from two LA-positive patients were used as the test samples No.1 and No.2, as well as normal plasma. Further, used were 38N2351 as bovine-derived serum, 35K3466 column purified product as poultry-derived serum, 1910DM as mouse-derived serum, and 9M0644 as horse-derived serum. All of these serums are available from GIBCO.

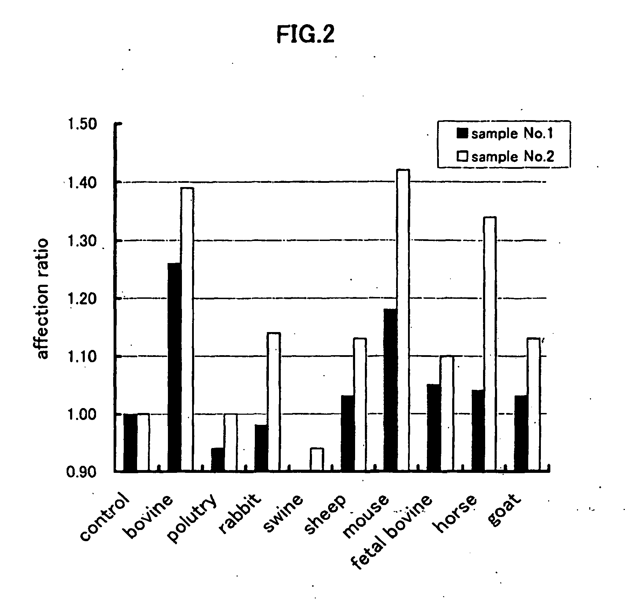

As is the case with Example 2, coagulation times of the samples were measured by using the automatic blood coagulation analyzer "Coagrex-800" (available from Shimadzu Corp.). The measurement results are shown in Table 4 and FIG. 2. In Table 4 and FIG. 2, coagulation times of the samples treated with the second coagulation time reagent free of the serums from the vertebrate animals are also shown, as second coagulation times for control groups. Further, Table 5 shows the affecting ratio calculated according to the above equation in respective cases where the serums are used.

Table 4

| sample | | serum | | | | | | | | | HBR |
|---|---|---|---|---|---|---|---|---|---|---|---|
| | contorl | bovine | polutry | rabbit | swine | sheep | mouse | fetal bovine | horse | goat | |
| normal plasma | 26.0 | 26.3 | 27.0 | 26.4 | 29.2 | 26.8 | 27.8 | 26.0 | 27.4 | 26.8 | 26.3 |
| sample No.1 | 52.0 | 41.3 | 55.5 | 53.0 | 57.6 | 50.5 | 44.1 | 49.5 | 49.9 | 50.5 | 42.1 |
| sample No.2 | 63.6 | 45.9 | 63.6 | 56.0 | 67.4 | 56.4 | 44.8 | 57.8 | 47.3 | 56.2 | 45.3 |

Table 5

| sample | | serum | | | | | | | | | HBR |
|---|---|---|---|---|---|---|---|---|---|---|---|
| | control | bovine | polutry | rabbit | swine | sheep | mouse | fetal bovine | horse | goat | |
| sample No.1 | 1.00 | 1.26 | 0.94 | 0.98 | 0.90 | 1.03 | 1.18 | 1.05 | 1.04 | 1.03 | 1.24 |
| sample No.2 | 1.00 | 1.39 | 1.00 | 1.14 | 0.94 | 1.13 | 1.42 | 1.10 | 1.34 | 1.13 | 1.40 |

As is obvious from Tables 4 and 5, both the samples No. 1 and No. 2, which are derived from the LA-positive patients, showed the affecting ratio of not smaller than 1 in case of being treated with the reagent containing the serums derived from mammals other than humans. However, the sample No. 1 having a low sensitivity to the poultry-derived serum showed an affecting ratio of not larger than 1, in case of being treated with the reagent containing the poultry-derived serum. Further, both the samples No. 1 and No. 2 showed a low affecting ratio, in case of being treated with the reagent containing the swine-derived serum, although the affecting ratio exceeded 1.

Example 4

Immunological method using non-human-derived antibodies or the like such as HBR:

(LA measuring method by ELISA system)

Prepared was a reagent containing 20 µg/mL in HBR concentration by diluting HBR (Scantibodies Laboratory, Inc.) with 0.1% NaN$_3$ and 0.1M phosphoric acid buffer (pH=7.5). 100 µL of the reagent was distributed into each well of a 96-well microplate. The microplate was put in a refrigerator overnight for sensitization. Thereafter, the microplate was rinsed with a liquid containing 0.05% Tween20 and PBS(pH=7.0), followed by addition of 50mM PBS (pH=7.4) containing 1% of bovine serum albumin (BSA). 100 µL of plasma from an APS patient or an LA-positive patient which was diluted by 20 times with 50mM PBS (pH=7.4) containing 1% of BSA was added, as a sample, to the microplate to which the HBR was fixed. The admixtures were reacted at a room temperature for 30 minutes to bind the sample to the HBR. After the plate was rinsed, 0.1 µg/mL of HBR to which biotin was bonded was added for reaction at a room temperature for 30 minutes. Thus, a complex of HBR/sample/biotin-bonded HBR was produced. After the biotin-bonded HBR which did not constitute the complex was removed by rinsing, 100µL of peroxidase (10mU/mL) labeled with strepto

**EP 1 469 317 B1**

avidin was added to react at a room temperature for 30 minutes. Thus, the biotin-bonded HBR of the complex was labeled by the labeled peroxidase. After peroxidase which did not bind to the biotin-bonded HBR was removed by rinsing, a liquid containing o-phenylenediamine (OPD) as a substrate was added for enzyme reaction at a room temperature for 15 minutes. The enzyme reaction was stopped by adding 100 μL of a 2N sulfuric acid. The absorbance of the reaction product was measured at a wavelength of 492nm (primary wavelength) and a wavelength of 690nm (secondary wavelength) with use of the spectrophotometer. The measurement results are shown in Table 6.

Table 6

| Sample | Absorbance (490/690nm) |
|---|---|
| Normal plasma 1 | 0. 0 4 3 |
| Normal plasma 2 | 0.104 |
| LA-positive plasma 1 | 1.813 |
| LA-positive plasma 2 | 3.452 |
| LA-positive plasma 3 | 2.896 |
| LA-positive plasma 4 | 1.308 |
| LA-positive plasma 5 | 2.558 |
| Heparinized plasma 1 | 0.230 |
| Heparinized plasma 2 | 0.073 |
| Warfarinized plasma 1 | 0.403 |
| Warfarinized plasma 2 | 0.098 |
| Factor VIII deficiency | 0.112 |
| FactorIX deficiency | 0.329 |

As is obvious from Table 6, the absorbance of the plasma containing human LA was from 1.308 to 3.452, whereas the absorbance of the normal plasmas, the heparinized plasmas, the warfarinized plasmas, the plasmas from the patients having the factorVIII deficiency and the factorIX deficiency fell in the range from 0.084 to 0.403. The results reveal that HBR specifically binds anti-phospholipid antibodies, particularly LA. Thus, LA is quantitatively determined by utilizing the relation between the absorbance and the quantity of LA.

**Claims**

1. A reagent for measuring a coagulation time, said reagent comprising:

   a composition for coagulation in vitro, including calcium ions, a phospholipid, and at least one component selected from the group consisting of activator, viper venom, and tissue factor; and
   at least one component capable of capturing anti-phospholipid antibody selected from the group consisting of antibodies, plasma, serum, and immunoglobulin derived from vertebrate animals other than human.

2. The reagent according to claim 1, wherein the animals are selected from the mammal other than human and swine.

3. The reagent according to claim 1 or 2, wherein the activator is at least one selected from the group consisting of ellagic acid, kaolin, silica, and celite.

4. The reagent for measuring a coagulation time, wherein the coagulation time reagent comprises a first preparatory reagent and a second preparatory reagent, wherein the first preparatory reagent contains the phospholipid;

   the component capable of capturing anti-phospholipid antibody selected from the group consisting of antibodies, plasma, serum and immunoglobin derived from vertebrate animals other than human; and
   the component selected from the group consisting of activator, viper venom, and tissue factor,

and wherein the second preparatory reagent contains calcium ions

5. A reagent kit for detecting anti-phospholipid antibody comprising:

a first coagulation time reagent containing a composition for coagulation in vitro; and at least one component capable of capturing anti-phospholipid antibody selected from the group consisting of antibodies, plasma, serum, and immunoglobulin derived from vertebrate animals other than human, the composition for coagulation including calcium ions, a phospholipid, and at least one component selected from the group consisting of activator, viper venom, and tissue factor; and
a second coagulation time reagent containing a composition for coagulation in vitro, including calcium ions, a phospholipid and at least one component selected from the group consisting of activator, viper venom, and tissue factor, in the absence of the component capable of capturing anti-phospholipid antibody.

6. The reagent kit for detecting anti-phospholipid antibody according to claim 5, wherein the constitution of the composition for coagulation in the first coagulation time reagent is the same as the composition for coagulation in the second coagulation time reagent.

7. The reagent kit for detecting anti-phospholipid antibody according to claim 5 or 6, wherein the anti-phospholipid antibody is human lupus anticoagulant.

8. The reagent kit for detecting anti-phospholipid antibody comprising a first coagulation time reagent which comprises a first preparatory reagent and a second preparatory reagent, and a second coagulation time reagent which comprises a third preparatory reagent and a fourth preparatory reagent,
the first preparatory reagent containing the phospholipid; the component capable of capturing anti-phospholipid antibody selected from the group consisting of antibodies, plasma, serum and immunoglobin derived from vertebrate animals other than human ; and the component selected from the group consisting of activator, viper venom, and tissue factor,
the second preparatory reagent containing calcium ions,
the third preparatory reagent containing the phospholipid, and the component selected from the group consisting of activator, viper venom, and tissue factor, and
the fourth preparatory reagent containing calcium ions.

9. A method for detecting anti-phospholipid antibody comprising the steps of:

contacting a sample with a first coagulation time reagent and a second coagulation time reagent respectively; and comparing a first coagulation time of the sample exposed to the first coagulation time reagent, with a second coagulation time of the sample exposed to the second coagulation time reagent,
wherein the first coagulation time reagent contains a composition for coagulation in vitro; and at least one component capable of capturing anti-phospholipid antibody selected from the group consisting of antibodies, plasma, serum, and immunoglobulin derived from vertebrate animals other than human, the composition for coagulation including calcium ions, a phospholipid, and at least one component selected from the group consisting of activator, viper venom, and tissue factor, and
the second coagulation time reagent contains a composition for coagulation in vitro, including calcium ions, a phospholipid, and at least one component selected from the group consisting of activator, viper venom, and tissue factor, in the absence of the component capable of capturing anti-phospholipid antibody.

10. The method for detecting anti-phospholipid antibody according to claim 9, further comprising judging that the sample contains the anti-phospholipid antibody if the first coagulation time is significantly different from the second coagulation time.

11. The method for detecting anti-phospholipid antibody according to claim 9, further comprising judging that the sample contains the anti-phospholipid antibody if the sample has an affecting ratio not smaller than 1.2, the affecting ratio being calculated from the following equation using the first coagulation time and the second coagulation time:

12. The method for detecting anti-phospholipid antibody according to any of claims 9 to 11, wherein the first coagulation time reagent is added to the sample so that the total amount of the component capable of capturing anti-phospholipid antibody relative to 1 ml of the sample ranges from 0.1 to 50 mg.

**Patentansprüche**

1. Reagens zur Messung einer Koagulationszeit, wobei das Reagens folgendes umfaßt:

    eine Zusammensetzung zur in-vitro-Koagulation, einschließend Calciumionen, ein Phospholipid und wenigstens eine Komponente ausgewählt aus der Gruppe bestehend aus Aktivator, Giftschlangenvenum und Gewebefaktor; und
    wenigstens eine Komponente, die einen anti-Phospholipid-Antikörper einfangen kann, ausgewählt aus der Gruppe bestehend aus Antikörpern, Plasma, Serum und Immunglobulin, die von Wirbeltieren stammen, die verschieden vom Menschen sind.

2. Reagens gemäß Anspruch 1, worin die Tiere von Säugetieren ausgewählt sind, die vom Menschen und Schwein verschieden sind.

3. Reagens gemäß Anspruch 1 oder 2, worin der Aktivator wenigstens einer ist, der aus der Gruppe ausgewählt ist, die aus Ellagsäure, Kaolin, Kieselsäure und Celit besteht.

4. Reagens zur Messung einer Koagulationszeit, wobei das Koagulationszeitreagens ein erstes Vorbereitungsreagens und ein zweites Vorbereitungsreagens umfaßt,
    worin das erste Vorbereitungsreagens das Phospholipid, die Komponente, die einen anti-Phospholipid-Antikörper einfangen kann, ausgewählt aus der Gruppe bestehend aus Antikörpern, Plasma, Serum und Immunglobulin, die von Wirbeltieren stammen, die verschieden vom Menschen sind, und die Komponente ausgewählt aus der Gruppe bestehend aus Aktivator, Giftschlangenvenum und Gewebefaktor enthält, und
    worin das zweite Vorbereitungsreagens Calciumionen enthält.

5. Reagenskit zum Nachweis von anti-Phospholipid-Antikörper, umfassend:

    ein erstes Koagulationszeitreagens, das eine Zusammensetzung zur in-vitro-Koagulation und wenigstens eine Komponente, die einen anti-Phospholipid-Antikörper einfangen kann, ausgewählt aus der Gruppe bestehend aus Antikörpern, Plasma, Serum und Immunglobulin, die von Wirbeltieren stammen, die verschieden vom Menschen sind, enthält, wobei die Zusammensetzung zur Koagulation Calciumionen, ein Phospholipid und wenigstens eine Komponente ausgewählt aus der Gruppe bestehend aus Aktivator, Giftschlangenvenum und Gewebefaktor einschließt; und
    ein zweites Koagulationszeitreagens, das eine Zusammensetzung zur in-vitro-Koagulation in Abwesenheit der Komponente, die einen anti-Phospholipid-Antikörper einfangen kann, enthält, die Calciumionen, ein Phospholipid und wenigstens eine Komponente ausgewählt aus der Gruppe bestehend aus Aktivator, Giftschlangenvenum und Gewebefaktor einschließt.

6. Reagenskit zum Nachweis von anti-Phospholipid-Antikörper gemäß Anspruch 5, worin die Zusammensetzung der Zusammensetzung zur Koagulation im ersten Koagulationszeitreagens die gleiche ist wie für die Zusammensetzung zur Koagulation im zweiten Koagulationszeitreagens.

7. Reagenskit zum Nachweis von anti-Phospholipid-Antikörper gemäß Anspruch 5 oder 6, worin der anti-Phospholipid-Antikörper der humane Lupus-Antikoagulant ist.

8. Reagenskit zum Nachweis von anti-Phospholipid-Antikörper, umfassend:

    ein erstes Koagulationszeitreagens, das ein erstes Vorbereitungsreagens und ein zweites Vorbereitungsreagens umfaßt, und ein zweites Koagulationszeitreagens, das ein drittes Vorbereitungsreagens und ein viertes Vorbereitungsreagens umfaßt, wobei
    das erste Vorbereitungsreagens das Phospholipid, die Komponente, die einen anti-Phospholipid-Antikörper einfangen kann, ausgewählt aus der Gruppe bestehend aus Antikörpern, Plasma, Serum und Immunglobulin, die von Wirbeltieren stammen, die verschieden vom Menschen sind, und die Komponente ausgewählt aus der Gruppe bestehend aus Aktivator, Giftschlangenvenum und Gewebefaktor enthält,
    das zweite Vorbereitungsreagens Calciumionen enthält,
    das dritte Vorbereitungsreagens das Phospholipid und die Komponente ausgewählt aus der Gruppe bestehend aus Aktivator, Giftschlangenvenum und Gewebefaktor enthält, und
    das vierte Vorbereitungsreagens Calciumionen enthält.

**9.** Verfahren zum Nachweis von anti-Phospholipid-Antikörper, umfassend die Schritte:

Kontaktieren einer Probe mit einem ersten Koagulationszeitreagens bzw. einem zweiten Koagulationszeitreagens; und

Vergleichen einer ersten Koagulationszeit der Probe, die dem ersten Koagulationszeitreagens ausgesetzt war, mit einer zweiten Koagulationszeit der Probe, die dem zweiten Koagulationszeitreagens ausgesetzt war, worin das erste Koagulationszeitreagens eine Zusammensetzung zur in-vitro-Koagulation und wenigstens eine Komponente, die einen anti-Phospholipid-Antikörper einfangen kann, ausgewählt aus der Gruppe bestehend aus Antikörpern, Plasma, Serum und Immunglobulin, die von Wirbeltieren stammen, die verschieden vom Menschen sind, enthält, wobei die Zusammensetzung zur Koagulation Calciumionen, ein Phospholipid und wenigstens eine Komponente ausgewählt aus der Gruppe bestehend aus Aktivator, Giftschlangenvenum und Gewebefaktor einschließt, und

das zweite Koagulationszeitreagens eine Zusammensetzung zur in-vitro-Koagulation in Abwesenheit der Komponente, die einen anti-Phospholipid-Antikörper einfangen kann, enthält, die Calciumionen, ein Phospholipid und wenigstens eine Komponente ausgewählt aus der Gruppe bestehend aus Aktivator, Giftschlangenvenum und Gewebefaktor einschließt.

**10.** Verfahren zum Nachweis von anti-Phospholipid-Antikörper gemäß Anspruch 9, ferner umfassend das Beurteilen, daß die Probe den anti-Phospholipid-Antikörper enthält, falls die erste Koagulationszeit signifikant von der zweiten Koagulationszeit verschieden ist.

**11.** Verfahren zum Nachweis von anti-Phospholipid-Antikörper gemäß Anspruch 9, ferner umfassend das Beurteilen, daß die Probe den anti-Phospholipid-Antikörper enthält, falls die Probe ein Beeinträchtigungsverhältnis aufweist, das nicht kleiner als 1,2 ist, wobei das Beeinträchtigungsverhältnis gemäß der folgenden Gleichung unter Verwendung der ersten Koagulationszeit und der zweiten Koagulationszeit berechnet wird:

**12.** Verfahren zum Nachweis von anti-Phospholipid-Antikörper gemäß einem der Ansprüche 9 bis 11, worin das erste Koagulationszeitreagens zu der Probe zugegeben wird, so daß die Gesamtmenge der Komponente, die anti-Phospholipid-Antikörper einfangen kann, relativ zu 1 ml der Probe im Bereich von 0,1 bis 50 mg vorliegt.

**Revendications**

**1.** Réactif pour mesurer un temps de coagulation, ledit réactif comprenant :

une composition pour coagulation *in vitro,* incluant des ions calcium, un phospholipide, et au moins un composant choisi dans le groupe constitué par un activateur, le venin de vipère, et un facteur tissulaire ; et
au moins un composant capable de capturer un anticorps anti-phospholipide choisi dans le groupe constitué par les anticorps, le plasma, le sérum, et une immunoglobuline dérivés d'animaux vertébrés autres que l'humain.

**2.** Réactif selon la revendication 1, dans lequel les animaux sont choisis parmi les mammifères autres que l'humain et le porc.

**3.** Réactif selon la revendication 1 ou 2, dans lequel l'activateur est au moins un activateur choisi dans le groupe constitué par l'acide ellagique, le kaolin, la silice, et la célite.

**4.** Réactif pour mesurer un temps de coagulation, dans lequel le réactif de temps de coagulation comprend un premier réactif préparatoire et un deuxième réactif préparatoire, dans lequel le premier réactif préparatoire contient le phospholipide ;

le composant capable de capturer un anticorps anti-phospholipide choisi dans le groupe constitué par les anticorps, le plasma, le sérum et une immunoglobuline dérivés d'animaux vertébrés autres que l'humain ; et
le composant choisi dans le groupe constitué par un activateur, le venin de vipère, et un facteur tissulaire, et dans lequel le deuxième réactif préparatoire contient des ions calcium.

**5.** Kit de réactifs pour détecter un anticorps anti-phospholipide comprenant :

un premier réactif de temps de coagulation contenant une composition pour coagulation *in vitro ;* et au moins un composant capable de capturer un anticorps anti-phospholipide choisi dans le groupe constitué par les anticorps, le plasma, le sérum, et une immunoglobuline dérivés d'animaux vertébrés autres que l'humain, la composition pour coagulation incluant des ions calcium, un phospholipide, et au moins un composant choisi dans le groupe constitué par un activateur, le venin de vipère, et un facteur tissulaire ; et

un deuxième réactif de temps de coagulation contenant une composition pour coagulation *in vitro,* incluant des ions calcium, un phospholipide et au moins un composant choisi dans le groupe constitué par un activateur, le venin de vipère, et un facteur tissulaire, en l'absence du composant capable de capturer un anticorps anti-phospholipide.

6. Kit de réactifs pour détecter un anticorps anti-phospholipide selon la revendication 5, dans lequel la constitution de la composition pour coagulation dans le premier réactif de temps de coagulation est identique à la composition pour coagulation dans le deuxième réactif pour temps de coagulation.

7. Kit de réactifs pour détecter un anticorps anti-phospholipide selon la revendication 5 ou 6, dans lequel l'anticorps anti-phospholipide est un anticoagulant de lupus humain.

8. Kit de réactifs pour détecter un anticorps anti-phospholipide comprenant un premier réactif de temps de coagulation qui comprend un premier réactif préparatoire et un deuxième réactif préparatoire, et un deuxième réactif de temps de coagulation qui comprend un troisième réactif préparatoire et un quatrième réactif préparatoire,

le premier réactif préparatoire contenant le phospholipide ; le composant capable de capturer un anticorps anti-phospholipide choisi dans le groupe constitué par les anticorps, le plasma, le sérum, et une immunoglobuline dérivés d'animaux vertébrés autres que l'humain ; et le composant choisi dans le groupe constitué par un activateur, le venin de vipère, et un facteur tissulaire,

le deuxième réactif préparatoire contenant des ions calcium,

le troisième réactif préparatoire contenant le phospholipide, et le composant choisi dans le groupe constitué par un facteur de contact, le venin de vipère, et un facteur tissulaire, et

le quatrième réactif préparatoire contenant des ions calcium.

9. Procédé de détection d'un anticorps anti-phospholipide comprenant les étapes de :

mise en contact d'un échantillon avec respectivement, un premier réactif de temps de coagulation et un deuxième réactif de temps de coagulation ; et

comparaison d'un premier temps de coagulation de l'échantillon exposé au premier réactif de temps de coagulation, avec un deuxième temps de coagulation de l'échantillon exposé au deuxième réactif de temps de coagulation,

dans lequel le premier réactif de temps de coagulation contient une composition pour coagulation *in vitro ;* et au moins un composant capable de capturer un anticorps anti-phospholipide choisi dans le groupe constitué par les anticorps, le plasma, le sérum, et une immunoglobuline dérivés d'animaux vertébrés autres que l'humain, la composition pour coagulation incluant des ions calcium, un phospholipide, et au moins un composant choisi dans le groupe constitué par un activateur, le venin de vipère, et un facteur tissulaire, et

le deuxième réactif de temps de coagulation contient une composition pour coagulation in vitro, incluant des ions calcium, un phospholipide, et au moins un composant choisi dans le groupe constitué par un activateur, le venin de vipère, et un facteur tissulaire, en l'absence du composant capable de capturer l'anticorps anti-phospholipide.

10. Procédé de détection d'un anticorps anti-phospholipide selon la revendication 9, comprenant en outre juger que l'échantillon contient l'anticorps anti-phospholipide si le premier temps de coagulation est significativement différent du deuxième temps de coagulation.

11. Procédé de détection d'un anticorps anti-phospholipide selon la revendication 9, comprenant en outre juger que l'échantillon contient l'anticorps anti-phospholipide si l'échantillon présente un rapport d'influence non inférieur à 1,2, le rapport d'influence étant calculé à partir de l'expression suivante en utilisant le premier temps de coagulation et le deuxième temps de coagulation :

12. Procédé de détection d'un anticorps anti-phospholipide selon l'une quelconque des revendications 9 à 11, dans lequel le premier réactif de temps de coagulation est ajouté à l'échantillon de sorte que la quantité totale du composant capable de capturer l'anticorps anti-phospholipide par rapport à 1 ml de l'échantillon varie de 0,1 à 50 mg.

EP 1 469 317 B1

# FIG.1

Legend:
- · · ◆ · · normal plasma 1
- · · ○ · · normal plasma 2
- —○— LA-positive plasma 1
- —△— LA-positive plasma 2
- —▲— LA-positive plasma 3
- —■— LA-positive plasma 4
- —◆— LA-positive plasma 5
- — ✕ · · Heparinized plasma 1
- — ✕ · · Heparinized plasma 2
- — + · · Warfarinized plasma 1
- — — · Warfarinized plasma 2
- — ◻ · · Factor VIII deficiency
- — ◪ · · Factor IX deficiency

Y-axis: coagulation time (sec)
X-axis: concentration of HBR (0, 2, 5, 10, 20, 30)

## FIG.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Guidebook on Laboratory Tests. Kanehara & Co., Ltd, 442
- **V.CHANTARANGKUL et al.** *Thromb. Res.,* 1992, vol. 67, 355-365
- **E.ROSNER et al.** *Thromb. Haemost.,* 1987, vol. 57, 144-149
- **BRANDT et al.** the International Society on Thrombosis and Hemostasis. *Thromb Haemost,* 1995, vol. 74 (4), 1185-90
- *Arthritis Rheum,* 1999, vol. 42 (7), 1309-11
- *Arthritis Rheum,* 2000, vol. 43 (9), 1982-93